# EUROPÄISCHE PATENTSCHRIFT

(11) **EP 1 863 843 B1**
(45) Veröffentlichungstag und Bekanntmachung des Hinweises auf die Patenterteilung: **02.08.2017**
(21) Anmeldenummer: 06706920.3
(22) Anmeldetag: 14.02.2006
(51) Int. Cl.: C07K 14/78, A61L 15/42, A61L 15/32

(54) **VERFAHREN ZUR REINIGUNG VON MARINEM KOLLAGEN SOWIE DESSEN VERARBEITUNG ZU PORÖSEN SCHWÄMMEN**
METHOD FOR CLEANING MARINE COLLAGEN AND THE TREATMENT THEREOF TO FORM POROUS SPONGES
PROCEDE DE PURIFICATION DE COLLAGENE MARIN ET TRANSFORMATION DE CE COLLAGENE EN EPONGES POREUSES

(30) Priorität: 24.02.2005 DE 102005008416
(43) Veröffentlichungstag der Anmeldung: 12.12.2007
(73) Patentinhaber: KliniPharm GmbH, 65760 Eschborn (DE)
(72) Erfinder: ALUPEI, Corneliu, Iulian, 56564 Neuwied (DE); RUTH, Peter, 56581 Melsbach (DE); ROHRER, Christian, 4020 Linz (AT); SCHATTON, Wolfgang, 60318 Frankfurt (DE)
(74) Vertreter: Müller, Claudia
(86) Internationale Anmeldenummer: PCT/EP2006/001313
(87) Internationale Veröffentlichungsnummer: WO 2006/089660

(56) Entgegenhaltungen:
- WO-A-98/00180
- WO-A-2004/026200
- WO-A2-01/64046
- US-A- 5 863 984
- SWATSCHEK DIETER ET AL: "Microparticles derived from marine sponge collagen (SCMPs): Preparation, characterization and suitability for dermal delivery of all-trans retinol." EUROPEAN JOURNAL OF PHARMACEUTICS AND BIOPHARMACEUTICS, Bd. 54, Nr. 2, September 2002 (2002-09), Seiten 125-133, XP004377354 ISSN: 0939-6411
- SWATSCHEK DIETER ET AL: "Marine sponge collagen: Isolation, characterization and effects on the skin parameters surface-pH, moisture and sebum" EUROPEAN JOURNAL OF PHARMACEUTICS AND BIOPHARMACEUTICS, Bd. 53, Nr. 1, Januar 2002 (2002-01), Seiten 107-113, XP004331337 ISSN: 0939-6411

## Beschreibung

Die vorliegende Erfindung betrifft ein Verfahren zur Reinigung von marinem Kollagen, insbesondere zur Verbesserung des Geruchs und des Aussehens von marinem Kollagen.

Die Erfindung betrifft ferner ein Verfahren zur Herstellung von porösen Schwämmen aus marinem Kollagen, die insbesondere für medizinische Zwecke geeignet sind.

Kollagen ist ein biologisch abbaubares wie auch biokompatibles Protein, das als Ausgangsmaterial für vielfältige Anwendungen in der Lebensmittelindustrie, in der pharmazeutischen und kosmetischen Industrie sowie in der Medizin verwendet wird.

Unter "marinem Kollagen" wird Kollagen verstanden, das aus marinen, also im Meer lebenden Organismen isoliert wurde, insbesondere aus Vertretern des Stamms der Porifera, vorzugsweise aus *Chondrosia reniformis.*

Zahlreiche Kollagenprodukte sind bekannt und werden in der Medizin angewendet. Zu diesen Produkten gehören beispielsweise Schwämme, Fasern oder Membranen.

Diese Produkte werden weit überwiegend aus Kollagen hergestellt, das aus dem Bindegewebe, der Haut, den Knochen oder den Sehnen von Säugetieren gewonnen wurde, beispielsweise von Rindern, Pferden oder Schweinen. Die wesentlichen Nachteile dieser Produkte sind darin zu sehen, dass
- meistens ein Jungtier als Quelle für das Kollagen dienen muss, um eine hinreichende Ausbeute an Kollagen zu erhalten;
- das erhaltene Kollagen meistens sowohl in sauren wie auch in basischen Medien löslich ist, was eine zusätzliche Vernetzungsreaktion (physikalisch durch Hitzebehandlung oder chemisch durch die Verwendung bifunktionaler Substanzen) erforderlich macht, um die mechanischen Eigenschaften des Kollagens und seine Stabilität in flüssigen Medien zu verbessern;
- das Risiko einer Kontamination mit der Bovinen Spongiformen Encephalopathie (BSE) besteht.

Als Alternative zur Gewinnung von Kollagen aus Säugetieren wird in WO 01/64046 ein Verfahren zur Isolierung von Kollagen aus marinen Schwämmen der Gattung *Chondrosia reniformis* (Porifera, Demospongiae) beschrieben.

Bei diesem Verfahren wird frisches Ausgangsschwammmaterial in Alkohol eingelegt, anschließend mit Wasser gewaschen und mit einem Extraktionsmittel, vorzugsweise bei einem pH-Wert von 7-12, versetzt. Der resultierende Kollagenextrakt wird durch Erhöhung des pH der Suspension auf einen Wert von 8-11, Rühren, Zentrifugieren, anschließende Verringerung des pH-Wertes des Überstands, sowie Zentrifugieren und Isolieren des Präzipitats aufarbeitet.

Das mit diesem Verfahren erhältliche Schwammkollagen weist jedoch Nachteile auf, die im Wesentlichen darin bestehen, dass
- die Kollagenlösung ein schmutziges Aussehen hat,
- das Kollagen mikrobiologisch unrein ist, und
- Produkte aus diesem Schwammkollagen einen unangenehmen Geruch aufweisen.

Die der vorliegenden Erfindung zugrunde liegende Aufgabe bestand daher darin, die erwähnten Nachteile von marinem Kollagen zu beseitigen und Kollagen aus marinen Organismen bereitzustellen, das auch für die Herstellung von Produkten für medizinische Zwecke geeignet ist.

Die Aufgabe wird durch ein Verfahren gelöst, bei dem ein Kollagenpräzipitat durch chemische Behandlungsschritte gereinigt und dadurch in seinem Geruch und seinem Aussehen verbessert wird. Das gereinigte Kollagen kann nachfolgend durch Gefriertrocknung zu porösen Schwämmen verarbeitet werden.

Gemäß der vorliegenden Erfindung umfasst das Verfahren zur Reinigung von marinem Kollagen die Behandlung eines Kollagenpräzipitats, wie es beispielsweise nach dem in WO 01/64046 beschriebenen Verfahren erhalten werden kann, mit Wasserstoffperoxid in wässrigen Lösungen bei unterschiedlichen pH-Werten.

Zu diesem Zweck sollte zunächst die Feuchtigkeit des Kollagenpräzipitats auf einen Gehalt von 70 bis 95 Gew.-%, vorzugsweise auf einen Gehalt von 80 bis 90 Gew.-%, eingestellt werden. Dies kann durch Auswringen des Kollagenpräzipitats unter Druck oder einer Zentrifugation erfolgen, um den Feuchtigkeitsgehalt des Kollagenpräzipitats erforderlichenfalls auf den gewünschten Gehalt zu reduzieren.

Das Kollagenpräzipitat wird dann in einer wässrigen R₂O₂-Lösung suspendiert, die einen H₂O₂-Gehalt von 0,1 bis 1 % (v/v), vorzugsweise von 0,5 % (v/v), aufweist. Dann wird der pH der Kollagensuspension auf einen Wert von zwischen 11 bis 13 eingestellt. Dadurch geht das Kollagen in Lösung.

Nach der Inkubation des Kollagens mit H₂O₂ unter alkalischen Bedingungen wird die Kollagenlösung filtriert, um unlösliche Bestandteile zu entfernen. Danach wird das Kollagen aus der Lösung ausgefällt, indem der Lösung ein geeignetes, mit Wasser mischbares, organisches Lösungsmittel zugesetzt wird.

Das Kollagenpräzipitat wird abfiltriert und dessen Feuchtigkeitsgehalt wieder auf 70 - 95 Gew.-%, vorzugsweise 80 bis 90 Gew.-%, eingestellt. Anschließend wird das Kollagen wieder in einer wässrigen H₂O₂-Lösung gelöst, die einen H₂O₂-Gehalt von 0,1 bis 1 % (v/v), vorzugsweise von 0,5 % (v/v), aufweist, und der pH dieser Kollagenlösung wird auf einen Wert von zwischen 5 bis 7 eingestellt, so dass das Kollagen dann für die Herstellung von Schwämmen, Membranen oder Fasern für medizinische Zwecke verwendet werden kann.

Die Einstellung des pH auf einen Wert von zwischen 11 bis 13 erfolgt vorzugsweise mit NaOH, kann aber auch mit anderen Alkali- oder Erdalkalihydroxiden wie beispielsweise KOH, Ca(OH)₂ oder Mg(OH)₂ erfolgen.

Als mit Wasser mischbares, organisches Lösungsmittel werden Ethanol und Isopropanol bevorzugt. Die Fällung des Kollagens erfolgt vorzugsweise bei einem Verhältnis von Wasser zu Ethanol, das zwischen 1:2 und 1:3 liegt.

Die Neutralisation der Kollagenlösung auf pH 5 bis 7 kann durch Reduktion des pH-Wertes mittels Zugabe organischer Säuren, z. B. Ameisensäure, Essigsäure, Zitronensäure, Ascorbinsäure, Propionsäure, Milchsäure, oder anorganischer Säuren wie Salzsäure, Phosphorsäure oder Schwefelsäure erfolgen. Vorzugsweise wird der pH-Wert durch Zugabe von 5 N Salzsäure eingestellt.

Die Vorteile des erfindungsgemäßen Verfahrens sind
- eine hohe Ausbeute bei der Protein-Extraktion;
- ein steriles Kollagen, das frei von Bakterien ist;
- ein BSE-freies Kollagen, da Porifera keine Nervenstrukturen haben,
- ein Kollagen mit einem ansprechenden Erscheinungsbild, das nicht schmutzig aussieht,
- ein Kollagen ohne unangenehmen Geruch;
- ein in sauren Medien nicht lösliches Kollagen, bei dem keine zusätzliche Vernetzungsreaktion erforderlich ist, um dessen mechanischen Eigenschaften und Stabilität in Flüssigkeiten zu verbessern;
- ein Kollagen, das für die Herstellung von Verbänden zur Wundheilung oder als Stütze bei der Geweberegeneration genutzt werden kann.

Aufgrund seiner Vorteile ist das mit dem erfindungsgemäßen Verfahren gereinigte Kollagen besonders gut für die Herstellung von Produkten für medizinische Zwecke geeignet.

Daher sind auch das mit dem erfindungsgemäßen Verfahren gereinigte Kollagen sowie dessen Verwendung zur Herstellung von porösen Schwämmen, Fasermaterial oder Membranen Gegenstand der Erfindung.

Die Erfindung betrifft daher auch Verfahren zur Herstellung von porösen Schwämmen aus dem gereinigten marinen Kollagen.

Poröse Kollagenschwämme können durch ein Verfahren hergestellt werden, bei dem eine nach dem erfindungsgemäßen Verfahren erhaltene Lösung des marinen Kollagens tiefgefroren und gefriergetrocknet wird, nachdem die Kollagenlösung entweder durch kräftiges Schütteln/Rühren aufgeschäumt oder die in der Kollagenlösung enthaltene Luft unter Vakuum entfernt wurde, um Schwämme mit kleineren Poren zu erhalten. Die Kollagenlösung enthält Kollagen vorzugsweise in einer Konzentration von 0,5 bis 5 Gew.-%.

Durch die Verwendung entsprechender Formen, in denen die Kollagenlösung bzw. der Kollagenschaum eingefroren wird, lassen sich poröse Kollagenschwämme beliebiger Gestalt herstellen.

Kollagenschwämme mit weiter verbesserten Eigenschaften, insbesondere im Hinblick auf ihre medizinische Verwendung, lassen sich durch eine Säurebehandlung der Kollagenschwämme erreichen, nachdem sie gefriergetrocknet wurden. Die Säurebehandlung erfolgt mit anorganischen Säuren, vorzugsweise durch Tauchen des Schwamms in eine 0,1 N HCl-Lösung. Danach kann der säurebehandelte Schwamm durch Tiefgefrieren und Gefriertrocknung, oder durch Eintauchen in Ethanol und Lufttrocknung dehydriert werden.

Mit dem erfindungsgemäßen Verfahren zur Herstellung von Kollagenschwämmen können auch poröse Kollagenschwämme mit antimikrobiellen Eigenschaften hergestellt werden. Dazu kann der Kollagensuspension vor dem Tiefgefrieren eine antimikrobiell wirksame Substanz zugesetzt werden. Eine bevorzugte antimikrobiell wirksame Substanz ist Silbersulfadiazin, die in einer Menge von 1 Gew.-%, bezogen auf die Kollagemmenge in der Suspension, zugesetzt wird.

Alternativ zu der vorhergehenden Vorgehensweise können die porösen Kollagenschwämme nach der Säurebehandlung in eine Lösung mit einer antimikrobiell wirksamen Substanz getaucht werden. Anschließend kann der Kollagenschwamm durch Gefriertrocknen oder, sofern die antimikrobiell wirksame Substanz nicht in Ethanol löslich ist, durch Eintauchen in Ethanol und anschließende Lufttrocknung dehydriert werden.

Gegenstand der Erfindung sind somit auch die mit den vorgenannten Verfahren hergestellten porösen Kollagenschwämme und deren Verwendung zur Herstellung von Produkten für medizinische Zwecke wie beispielsweise Wundauflagen.

### Beispiel 1

### Verfahren zur Reinigung von Schwammkollagen

Ein in einem sauren Medium (pH 3) ausgefälltes Kollagenpräzipitat, welches nach dem in WO 01/64046 beschriebenen Verfahren erhalten worden war, wurde durch Filtration vom Medium abgetrennt und unter Druck ausgewrungen, bis ein verbleibender Feuchtigkeitsgehalt von etwa 84 Gew.-% erreicht wurde. Die auf diese Weise erhaltenen Kollagenfasern wurden für die Lagerung bei einer Temperatur von etwa -20°C tiefgefroren.

121 Gramm der gefrorenen Kollagenfasern wurden unter zweistündigem Rühren in 1300 ml einer wässrigen 0,5 %-igen (v/v) H₂O₂-Lösung suspendiert. Dann wurde der pH der Lösung mittels einer 5 N Lösung NaOH auf einen Wert von 12,4 eingestellt, um die Kollagenfasern in Lösung zu bringen. Die resultierende Kollagenlösung wurde filtriert, um unlösliche Bestandteile zu entfernen, und anschließend unter kräftigem Rühren in 2600 ml Ethanol (Konz. 98 %) gegossen. Das dadurch ausgefällte Kollagen wies eine weiße oder leicht gelbliche Farbe sowie eine faserige Erscheinung auf.

Die Kollagenfasern wurden durch Filtration vom Medium befreit, unter Druck oder mittels Zentrifugation ausgewrungen, und anschließend unter Rühren in 300 ml einer wässrigen 0,5 %-gen (v/v) H₂O₂-Lösung homogen suspendiert. Der pH der Suspension wurde mit einer 5 N HCl-Lösung auf einen Wert von 6,5 eingestellt. Die Einstellung des pH-Wertes erfolgte unter kräftigem Rühren, um die Bildung von Faserpräzipitaten zu vermeiden. Auf diese Weise wurde eine sterile Kollagenlösung mit einer Kollagen-Konzentration von 2,8 Gew.-% erhalten.

Alle Verfahrensschritte wurden bei Raumtemperatur durchgeführt und sämtliche Objekte, die mit dem Kollagen in Kontakt kommen, wurden vor ihrer Verwendung mit einer 0,5 %-gen H₂O₂-Lösung gespült.

### Beispiel 2

### Herstellung von porösen Schwämmen mit gereinigtem Schwammkollagen

Eine Lösung von Schwammkollagen mit einer Kollagen-Konzentration von 2 Gew.-% und einem pH-Wert von 6,5, die nach einem Verfahren gemäß Beispiel 1 erhalten worden war, wurde durch kräftiges Rühren mit Hilfe eines Ultra Turrax aufgeschäumt. Der Kollagenschaum wurde in eine rechteckige Form aus Polypropylen oder Polystyrol gegossen. Die Höhen der Schaumschichten variierten zwischen 2 und 8 mm. Der Kollagenschaum wurde in der Form bei -40°C eingefroren und anschließend lyophilisiert.

### Beispiel 3

### Herstellung von Schwämmen auf Basis von ausgefälltem Schwammkollagen

Es wurden poröse Schwämme aus Schwammkollagen wie in Beispiel 2 beschrieben hergestellt. Anschließend wurden die gefriergetrockneten Schwämme einer 30-minütigen Säurebehandlung durch Eintauchen in eine 0,1 N HCl-Lösung zugeführt. Nach dieser Behandlung wurden die Schwämme mehrfach mit destilliertem Wasser gewaschen, bis die letzen Reste an Säure entfernt waren.

Diese Schwämme wurden im noch feuchten Zustand eingefroren und durch Lyophilisierung getrocknet.

## Patentansprüche

1. Verfahren zur Reinigung von marinem Kollagen, umfassend die Schritte:
- Einstellen des Feuchtigkeitsgehalts eines in einem sauren Medium ausgefällten Kollagenpräzipitats auf 95 bis 70 Gew.-%,
- Suspendieren des Kollagens in einer wässrigen H₂O₂-Lösung
- Solubilisieren des Kollagens durch Einstellen des pH der Kollagensuspension auf einen Wert von zwischen 11 bis 13;
- Ausfällen des Kollagens mit einem organischen, mit Wasser mischbaren Lösungsmittel,
- Abfiltrieren des Kollagens,
- Einstellen des Feuchtigkeitsgehalts des ausgefällten Kollagens auf 95 bis 70 Gew.-%,
- Resuspendieren des Kollagens in einer wässrigen H₂O₂-Lösung, und
- Einstellen des pH der resultierenden Kollagenlösung auf einen Wert von zwischen 5 bis 7.

2. Verfahren zur Herstellung von porösen Schwämmen aus marinem Kollagen, umfassend die Schritte:
- Einstellen des Feuchtigkeitsgehalts eines in einem sauren Medium ausgefällten Kollagenpräzipitats auf 95 bis 70 Gew.-%,
- Suspendieren des Kollagens in einer wässrigen H₂O₂-Lösung
- Solubilisieren des Kollagens durch Einstellen des pH der Kollagensuspension auf einen Wert von zwischen 11 bis 13;
- Ausfällen des Kollagens mit einem organischen, mit Wasser mischbaren Lösungsmittel,
- Abfiltrieren des Kollagens,
- Einstellen des Feuchtigkeitsgehalts des ausgefällten Kollagens auf 95 bis 70 Gew.-%,
- Resuspendieren des Kollagens in einer wässrigen H₂O₂-Lösung, und
- Einstellen des pH der resultierenden Kollagenlösung auf einen Wert von zwischen 5 bis 7,
- Aufschäumen der resultierenden Kollagenlösung oder Entfernen der in der Lösung des resultierenden Kollagens enthaltenen Luft unter Vakuum,
- Tiefgefrieren der aufgeschäumten oder entgasten Kollagenlösung; und
- Gefriertrocknen der tiefgefrorenen Kollagenlösung.

3. Verfahren nach Anspruch 1 oder 2, **dadurch gekennzeichnet, dass** der Feuchtigkeitsgehalt auf 90 bis 80 Gew.-% eingestellt wird.

4. Verfahren nach einem der vorangehenden Ansprüche, **dadurch gekennzeichnet, dass** die wässrige H₂O₂-Lösung einen H₂O₂-Gehalt von 0,1 bis 1,0 % (v/v), vorzugsweise von 0,5 % (v/v), aufweist.

5. Verfahren nach einem der vorangehenden Ansprüche, **dadurch gekennzeichnet, dass** das organische, mit Wasser mischbare Lösungsmittel aus der Gruppe ausgewählt ist, die Ethanol und Isopropanol umfasst.

6. Verfahren nach Anspruch 2, **dadurch gekennzeichnet, dass** die Kollagenlösung einen Kollagengehalt von 0,5 bis 5 Gew.-% aufweist.

7. Verfahren nach Anspruch 2 oder 6, **dadurch gekennzeichnet, dass** der Kollagenlösung vor dem Aufschäumen oder Entgasen ein antimikrobieller Wirkstoff zugesetzt wird.

8. Verfahren nach Anspruch 7, **dadurch gekennzeichnet, dass** die Kollagenlösung den antimikrobiellen Wirkstoff in einer Menge von 1 Gew.-%, bezogen auf den Kollagengehalt der Lösung, enthält.

9. Verfahren nach Anspruch 2 oder 6, **dadurch gekennzeichnet, dass** der Kollagenschwamm nach seiner Gefriertrocknung mit einer anorganischen Säure, vorzugsweise einer 0,1 N HCl-Lösung, behandelt und danach mit Wasser ausgewaschen wird, bis keine Säure mehr nachzuweisen ist.

10. Verfahren nach Anspruch 9, **dadurch gekennzeichnet, dass** der mit Säure behandelte Schwamm in die Lösung einer antimikrobiell wirksamen Substanz getaucht wird.

11. Verfahren nach Anspruch 10, **dadurch gekennzeichnet, dass** der Schwamm nach dem Tauchen durch Tiefgefrieren und Gefriertrocknung, oder durch Tauchen in Ethanol und Lufttrocknung dehydriert wird.

12. Verfahren nach einem der Ansprüche 7, 8, 10 oder 11, **dadurch gekennzeichnet, dass** es sich bei dem antimikrobiellen Wirkstoff um Silbersulfadiazin handelt.

13. Poröser Schwamm aus marinem Kollagen, **dadurch gekennzeichnet, dass** er nach einem Verfahren gemäß einem der Ansprüche 2 bis 12 hergestellt wurde.

14. Verwendung eines porösen Schwamms aus marinem Kollagen, der nach einem Verfahren gemäß einem der Ansprüche 2 bis 12 hergestellt wurde, zur Herstellung medizinischer Produkte.

## Claims

1. A method for purifying marine collagen, comprising the steps of:
- adjusting the moisture content of a collagen precipitate, which has been precipitated in an acid medium, to a range of 95 to 70% by weight,
- suspending the collagen in an aqueous H₂O₂ solution,
- solubilising the collagen by adjusting the pH of the collagen suspension to a value ranging from 11 to 13;
- precipitating the collagen with an organic, water-miscible solvent,
- filtering out the collagen,
- adjusting the moisture content of the precipitated collagen to a range of 95 to 70% by weight,
- resuspending the collagen in an aqueous H₂O₂ solution, and
- adjusting the pH of the resulting collagen solution to a value ranging from 5 to 7.

2. A method for producing porous sponges of marine collagen, comprising the steps of:
- adjusting the moisture content of a collagen precipitate, which has been precipitated in an acid medium, to a range of 95 to 70% by weight,
- suspending the collagen in an aqueous H₂O₂ solution,
- solubilising the collagen by adjusting the pH of the collagen suspension to a value ranging from 11 to 13;
- precipitating the collagen with an organic, water-miscible solvent,
- filtering out the collagen,
- adjusting the moisture content of the precipitated collagen to a range of 95 to 70% by weight,
- resuspending the collagen in an aqueous H₂O₂ solution, and
- adjusting the pH of the resulting collagen solution to a value ranging from 5 to 7,
- foaming the resulting collagen solution or removing the air contained in the solution of the resulting collagen in vacuo,
- deep-freezing the foamed or degassed collagen solution; and
- lyophilising the deep-frozen collagen solution.

3. The method according to claim 1 or 2, **characterised in that** the moisture content is adjusted to a moisture content of 90 to 80% by weight.

4. The method according to any one of the preceding claims, **characterised in that** the aqueous H₂O₂ solution has an H₂O₂ content of 0.1 to 1.0% (v/v), preferably of 0.5% (v/v).

5. The method according to any one of the preceding claims, **characterised in that** the organic, water-miscible solvent is selected from the group comprising ethanol and isopropanol.

6. The method according to claim 2, **characterised in that** the collagen solution has a collagen content of 0.5 to 5% by weight.

7. The method according to claim 2 or 6, **characterised in that** an antimicrobial active substance is added to the collagen solution prior to foaming or degassing.

8. The method according to claim 7, **characterised in that** the collagen solution contains the antimicrobial active substance in an amount of 1% by weight, relative to the collagen content of the solution.

9. The method according to claim 2 or 6, **characterised in that** the collagen sponge, subsequent to the lyophilisation thereof, is treated with an inorganic acid, preferably an 0.1 N HCl solution, and thereafter washed out with water until there is no longer any acid detectable.

10. The method according to claim 9, **characterised in that** the sponge treated with acid is immersed in the solution of an antimicrobially active substance.

11. The method according to claim 10, **characterised in that** the sponge, following immersion thereof, is dehydrated by deep-freezing and lyophilisation, or by immersing in ethanol and air-drying.

12. The method according to any one of claims 7, 8, 10 or 11 **characterised in that** the antimicrobial active substance is silver sulfadiazine.

13. Porous sponge of marine collagen, **characterised in that** it has been produced according to a method as defined in any one of claims 2 to 12.

14. Use of a porous sponge of marine collagen which has been produced according to a method as defined in any one of claims 2 to 12 for the production of medical products.

## Revendications

1. Procédé pour la purification de collagène marin, comprenant les étapes dans lesquelles :
- on règle la teneur en humidité d'un précipité de collagène précipité dans un milieu acide, à une valeur de 95 à 70 % en poids ;
- on met le collagène en suspension dans une solution aqueuse de H₂O₂ ;
- on solubilise le collagène en réglant le pH de la suspension de collagène à une valeur entre 11 et 13 ;
- on précipite le collagène avec un solvant organique miscible à l'eau ;
- on sépare le collagène par filtration ;
- on règle la teneur en humidité du collagène précipité à une valeur de 95 à 70 % en poids ;
- on remet le collagène en suspension dans une solution aqueuse de H₂O₂ ; et
- on règle le pH de la solution de collagène résultante à une valeur entre 5 et 7.

2. Procédé pour la fabrication d'éponges poreuses à partir de collagène marin, comprenant les étapes dans lesquelles :
- on règle la teneur en humidité d'un précipité de collagène précipité dans un milieu acide, à une valeur de 95 à 70 % en poids ;
- on met le collagène en suspension dans une solution aqueuse de H₂O₂ ;
- on solubilise le collagène en réglant le pH de la suspension de collagène à une valeur entre 11 et 13 ;
- on précipite le collagène avec un solvant organique miscible à l'eau ;
- on sépare le collagène par filtration ;
- on règle la teneur en humidité du collagène précipité à une valeur de 95 à 70 % en poids ;
- on remet le collagène en suspension dans une solution aqueuse de H₂O₂ ; et
- on règle le pH de la solution de collagène résultante à une valeur entre 5 et 7 ;
- on transforme en mousse la solution de collagène résultante ou on élimine sous vide l'air que contient la solution du collagène résultant ;
- on congèle la solution de collagène transformée en mousse ou dégazée ; et
- on lyophilise la solution de collagène congelée.

3. Procédé selon la revendication 1 ou 2, **caractérisé en ce qu'**on règle la teneur en humidité à une valeur de 90 à 80 % en poids.

4. Procédé selon l'une quelconque des revendications précédentes, **caractérisé en ce que** la solution aqueuse de H₂O₂ présente une teneur en H₂O₂ de 0,1 à 1,0 % (volume/volume), de préférence de 0,5 % (volume/volume).

5. Procédé selon l'une quelconque des revendications précédentes, **caractérisé en ce que** le solvant organique miscible à l'eau est choisi parmi le groupe qui comprend l'éthanol et l'isopropanol.

6. Procédé selon la revendication 2, **caractérisé en ce que** la solution de collagène présente une teneur en collagène de 0,5 à 5 % en poids.

7. Procédé selon la revendication 2 ou 6, **caractérisé en ce que** l'on ajoute à la solution de collagène, avant la transformation en mousse ou le dégazage, une substance active antimicrobienne.

8. Procédé selon la revendication 7, **caractérisé en ce que** la solution de collagène contient la substance active antimicrobienne en une quantité de 1 % en poids, rapporté à la teneur de la solution en collagène.

9. Procédé selon la revendication 2 ou 6, **caractérisé en ce que** l'on traite l'éponge de collagène après sa lyophilisation avec un acide inorganique, de préférence une solution de HCl 0,1 N, et on l'élimine ensuite par lavage avec de l'eau jusqu'à ce que l'on ne puisse plus déceler d'acide.

10. Procédé selon la revendication 9, **caractérisé en ce qu'**on plonge l'éponge traitée avec un acide dans la solution d'une substance à activité antimicrobienne.

11. Procédé selon la revendication 10, **caractérisé en ce qu'**on déshydrate l'éponge après l'immersion, par congélation et lyophilisation ou par immersion dans de l'éthanol et séchage à l'air.

12. Procédé selon l'une quelconque des revendications 7, 8, 10 ou 11, **caractérisé en ce que**, en ce qui concerne la substance active antimicrobienne, il s'agit de sulfadiazine d'argent.

13. Éponge poreuse obtenue à partir de collagène marin, **caractérisée en ce qu'**elle a été fabriquée conformément à un procédé selon l'une quelconque des revendications 2 à 12.

14. Utilisation d'une éponge poreuse obtenue à partir de collagène marin, qui a été fabriquée conformément à un procédé selon l'une quelconque des revendications 2 à 12, pour la préparation de produits médicaux.
